# FASCICULE DE BREVET EUROPEEN

(11) **EP 2 419 082 B1**
(45) Date de publication et mention de la délivrance du brevet: **22.01.2020**
(21) Numéro de dépôt: 09829861.5
(22) Date de dépôt: 21.12.2009
(51) Int. Cl.: A61K 9/06, A61K 31/05, A61K 31/592, A61K 31/593, A61K 31/7048, A61K 9/107

(54) **COMPOSITION PHARMACEUTIQUE TOPIQUE COMPRENANT UN PRINCIPE ACTIF SENSIBLE A L'EAU**
TOPISCHE PHARMAZEUTISCHE ZUSAMMENSETZUNG MIT EINEM WASSEREMPFINDLICHEN WIRKSTOFF
TOPICAL PHARMACEUTICAL COMPOSITION CONTAINING A WATER-SENSITIVE ACTIVE PRINCIPLE

(30) Priorité: 23.12.2008 US 193793 P
(43) Date de publication de la demande: 22.02.2012
(73) Titulaire: Galderma S.A., 6330 Cham (CH)
(72) Inventeur: NADAU-FOURCADE, Karine, F-06270 Villeneuve Loubet (FR); BARTHEZ, Nathalie, F-06700 Saint Laurentdu Var (FR); MAZEAU, Laëtitia, F-06800 Cagnes sur Mer (FR)
(74) Mandataire: Nederlandsch Octrooibureau
(86) Numéro de dépôt international: PCT/FR2009/052634
(87) Numéro de publication internationale: WO 2010/072958

(56) Documents cités:
- EP-A1- 0 462 723
- EP-A1- 0 462 723
- EP-A1- 0 462 723
- WO-A1-2004/093886
- WO-A1-2004/093886
- WO-A1-2005/044236
- WO-A1-2005/044236
- WO-A1-2005/044236
- WO-A1-2007/104895
- WO-A1-2007/104895
- WO-A1-2007/104895
- WO-A2-2004/037225
- WO-A2-2004/037225
- WO-A2-2004/037225
- WO-A2-2007/119028
- WO-A2-2007/119028
- WO-A2-2008/038147
- WO-A2-2008/038147
- WO-A2-2008/038147
- CA-A1- 2 500 907
- CA-A1- 2 500 907
- CA-A1- 2 500 907
- FR-A1- 2 850 575
- FR-A1- 2 850 575
- FR-A1- 2 850 575
- FR-A1- 2 909 556
- FR-A1- 2 909 556
- FR-A1- 2 909 556
- FR-A1- 2 912 655
- FR-A1- 2 912 655
- FR-A1- 2 912 655
- US-A1- 2002 155 073
- US-A1- 2002 155 073
- US-A1- 2002 155 073
- ANONYMOUS: "Phenols", INTERNET CITATION, 15 novembre 2011 (2011-11-15), pages 1-5, XP002663805, Extrait de l'Internet: URL:http://en.wikipedia.org/wiki/Phenols [extrait le 2011-11-15]
- "Phenols", Wikipedia, pages 1-5, XP002663805, Extrait de l'Internet: URL:http://en.wikipedia.org/wiki/Phenols [extrait le 2011-11-15]
- ANONYMOUS: "Phenols", INTERNET CITATION, 15 November 2011 (2011-11-15), pages 1-5, XP002663805, Retrieved from the Internet: URL:http://en.wikipedia.org/wiki/Phenols [retrieved on 2011-11-15]

## Description

L'invention se rapporte à une composition pharmaceutique topique comprenant en tant qu'actif pharmaceutique un composé sensible à l'eau, sous une forme solubilisée, dans un milieu physiologiquement acceptable, à son procédé de préparation, et à son utilisation en dermatologie.

Dans le domaine de la dermatologie et de la formulation de compositions pharmaceutiques, l'homme du métier est amené à réaliser des compositions qui doivent être stables physiquement et chimiquement. Elles doivent également permettre de libérer l'actif et de favoriser sa pénétration à travers les couches cutanées afin d'en améliorer son efficacité.

De nombreux actifs présentent la difficulté d'être très faiblement solubles dans les solvants cosmétiques ou pharmaceutiques couramment utilisés, notamment l'eau, et/ou sensible à un environnement aqueux, oxydant. Cette sensibilité à l'eau peut conduire à une instabilité chimique de l'actif et/ou à une cristallisation de l'actif initialement solubilisé. Cette sensibilité à l'eau limite donc leur formulation dans des compositions cosmétiques ou dermatologiques appliquées par voie topique.

Pour obtenir une stabilité physique et chimique d'une composition comprenant un actif sensible à l'eau, l'homme de l'art sera tenté d'utiliser des compositions anhydres ou à très fortes teneurs en phase grasse. Cela confère ainsi un aspect gras et parfois collant à la composition, d'où une mauvaise acceptabilité cosmétique. L'homme du métier sait également que la non observance du traitement prescrit pour des raisons invoquées précédemment est l'une des causes principales d'échec, notamment pour le traitement du psoriasis, l'article « patients with psoriasis and their compliance with medication » (Richards & all, J Am Acad Dermatol Oct 99, p581-583) indique que près de 40% des patients avec une maladie chronique telle que le psoriasis ne suivent pas leur traitement. Il a été démontré que l'adhésion du patient à son traitement est directement liée aux caractéristiques du véhicule de la composition appliquée. L'article « Patients with psoriasis prefer solution and foam vehicles : a quantitative assessment of vehicle préférence » (Housman & all; CUTIS, dec 2002 vol 70, p 327 à 332) indique que les patients psoriatiques préféreront une solution, une mousse, ou une émulsion légère, plutôt qu'un onguent, ou qu'une crème épaisse et grasse .

En effet, à la lecture de l'art antérieur, les compositions existantes, permettant de formuler sous forme solubilisée un actif sensible à l'eau, contiennent souvent un fort pourcentage de d'huile et souvent de vaseline pour favoriser l'occlusivité et la pénétration de l'actif. Elles présentent donc l'inconvénient d'être très grasses et collantes, ne favorisant pas ainsi le confort et la facilité d'application. Les autres types de compositions couramment rencontrées dans l'art antérieur contiennent un fort pourcentage de glycol propénétrant afin de favoriser la pénétration de l'actif mais sont souvent collantes et peuvent provoquer des problèmes d'intolérance. (« The critical role of the vehicle to therapeutic efficacy and patient compliance" Piacquadio & all, Journal of American Academy of dermatology, August 1998).
WO04093886 *décrit également des compositions topiques à base d'ivermectine.*

Afin de pallier à ce problème d'inconfort et de facilité d'application, l'homme de l'art recherche donc à formuler l'actif au sein d'un véhicule cosmétiquement acceptable en plus d'une efficacité pharmaceutique. Ce qui est généralement le cas des émulsions, contenant une phase aqueuse. Le problème principal à résoudre est donc de stabiliser l'actif sensible à l'eau et la composition malgré une présence d'eau au sein de la composition.
WO07119028 *décrit une composition comprenant une phase aqueuse et au moins une phase grasse comprenant de l'ivermectine.*

Les phénomènes de dégradation chimique et/ou de cristallisation de l'actif en présence d'eau ont pour conséquence une diminution ou une perte d'efficacité et une incertitude quant à la dose d'actif mise en œuvre lors de son utilisation, ce qui va à l'encontre de l'objectif recherché. En outre, cette dégradation de l'actif et/ou sa cristallisation peuvent modifier la stabilité globale des compositions ainsi que leur aspect.

Par actif sensible à la présence d'eau selon l'invention, on entend donc un actif instable chimiquement et/ou physiquement. Par instabilité chimique, on entend notamment une dégradation du principe actif. Par instabilité physique, on entend notamment une cristallisation ou précipitation de l'actif, ou encore une modification de sa couleur au sein de la composition.

Une composition stable physiquement selon l'invention est, par conséquent, une composition ne présentant aucune modification d'aspect macroscopique (séparation de phase, changement de couleur d'aspect, etc..) ni microscopique (recristallisation des actifs) après stockage aux températures de 25°C, 4°C et 40°C, pendant 2, 4, 8, 12 semaines.

Une composition stable chimiquement selon l'invention est, par conséquent, une composition dans laquelle la teneur en principe actif reste stable après trois mois à température ambiante et à 40 °C. Une teneur stable en principe actif signifie selon l'invention que la teneur présente très peu de variation par rapport à la teneur initiale, c'est-à-dire que la variation de teneur en principe actif au temps T ne doit pas être inférieure à 90% et plus particulièrement à 95% de la teneur initiale à T0.

Il existe donc le besoin de disposer d'une composition permettant de répondre à un ou plusieurs des aspects suivants : disposer d'une bonne stabilité de la formule au froid et à la chaleur, en particulier quant au maintien de la taille des globules et à l'absence de déphasage, avoir une bonne résistance de l'actif vis-à-vis des phénomènes d'oxydation, permettre une bonne stabilité chimique de l'actif et une bonne disponibilité de celui-ci pour la peau. Il est également utile de pouvoir disposer d'une composition autorisant une forte fraction volumique dispersée. Il est par ailleurs utile que le mode de préparation de telles compositions soit aisé et avantageux.

L'homme du métier souhaite donc améliorer ces paramètres par la présente invention.

D'autres paramètres sont également à prendre en compte par l'homme de l'art pour le choix des ingrédients d'une composition pharmaceutique. En effet, la composition pharmaceutique utilisable selon l'invention en tant que médicament, devra également être formulée en accord avec la pathologie à traiter.

A titre d'exemple non limitatif, une composition destinée à traiter l'acné, se devra d'être d'aspect cosmétique non gras, alors qu'une composition destiné à traiter la dermatite atopique devra être émolliente, hydratante, et pourra être plus riche en corps gras, tout en évitant l'aspect gras non cosmétique.

Le problème que se propose de résoudre ici la présente invention, est donc de concevoir une composition pharmaceutique aqueuse de type émulsion huile dans eau, stable physiquement et chimiquement, comprenant au moins un principe actif sensible à l'eau, sous forme solubilisée. Ladite composition étant différente d'un onguent. La composition selon l'invention peut donc se présenter sous différentes formes d'émulsions : émulsion sprayable, lotion, lait, crème épaisse de texture variable plus ou moins riche selon la pathologie à traiter. Elle doit également présenter une facilité d'utilisation et une cosméticité acceptable pour une application sur toutes les zones du corps pouvant être touchées par la pathologie.

Dans la présente invention, la demanderesse a montré de façon surprenante, qu'il était possible d'obtenir une composition de type émulsion huile dans eau, contenant un actif sensible à l'eau, solubilisé dans la phase grasse interne, stable physiquement et chimiquement, la dite composition comprenant :
- au moins un principe actif sensible à l'eau,
- une phase grasse contenant au moins une phase lipophile solvante de l'actif,
- au moins un polyol
- au moins 5 % d'eau,
caractérisée en ce qu'elle est topique et qu'elle comprend au moins un tensioactif de la famille des sucroesters.

Par forme solubilisée de l'actif, on entend une dispersion de l'actif à l'état moléculaire dans un liquide, aucune cristallisation de l'actif n'étant visible à l'œil nu ni même au microscope optique en polarisation croisée.

Dans un mode la composition comprend au moins un actif sensible à l'eau choisi parmi les dérivés de vitamines liposolubles comme ceux de la vitamine A (rétinol), E ou C et plus particulièrement ceux de la vitamine D, les lactones macrocyliques ou encore les dérivés phénoliques.

Les dérivés de Vitamine D présentent une instabilité connue dans les milieux contenant de l'eau et plus particulièrement aux pH acides. La plupart du temps, ces dérivés sont formulés dans des préparations anhydres grasses.

De même, les dérivés phénoliques présentent une oxydation très rapide en milieux aqueux même en présence d'antioxydants. Il est donc difficile de les formuler en présence d'eau.

Les lactones macrocycliques présentent, elles aussi, une instabilité connue dans les milieux aqueux, ce qui rend difficile leur formulation dans une émulsion huile dans eau.

Ces principes actifs présentent tous l'inconvénient d'être instables dans les milieux aqueux, et/ou sensibles aux pH acides et donc difficilement formulables dans une composition contenant de l'eau. Même solubilisés dans la phase huileuse d'une émulsion huile dans eau classique, ces principes actifs peuvent migrer vers la phase aqueuse, déstabilisant ainsi la composition chimiquement et/ou physiquement.

La composition selon l'invention comprend donc au moins un principe actif sensible à l'eau choisi parmi les lactones macrocycliques.

A titre de dérivés de vitamine D, on peut citer les composés choisis parmi le calcitriol, le calcipotriol et le {4-[6-Ethyl-4'-(1-ethyl-1-hydroxy-propyl)-2'-propyl-biphenyl-3-yloxymethyl]-2-hydroxymethyl-phenyl}-méthanol.

Également decrit est une composition comprenant entre 0,00001 et 0,1% d'au moins un dérivé de la vitamine D en poids par rapport au poids total de la composition, de préférence de 0.0001 à 0.1%. De manière préférentielle, la composition selon l'invention contient de 0.0001% à 0.05% de calcitriol, de préférence de 0.003% à 0.06%.

A titre de lactones macrocycliques utilisables selon l'invention, on peut citer les composés choisis parmi les avermectines, tel l'ivermectine, l'invermectine, l'avermectine, l'abamectine, la doramectine, l'eprinomectine et la sélamectine, l'aversectine B, AB ou C, l'émamectine B1a, l'émamectine B1b et leurs dérivés, ou la latidectine. Le composé de la famille des avermectines est de préférence l'ivermectine.

Dans les compositions selon l'invention, le composé de la famille des avermectines est présent à une concentration comprise entre 0,001 et 10 % en poids, par rapport au poids total de la composition le comprenant, de préférence entre 0,01 et 5 % en poids. En particulier la composition comprend 0,75%, 1%, 1,5% ou 2% d'ivermectine.

Par dérivé phénolique, on peut citer à titre non limitatif l'hydroquinone, le 4-hydroxyanisol, le rucinol, le monoethyl éther d'hydroquinone et le monobenzylether d'hydroquinone. De préférence, on utilise l'hydroquinone ou le rucinol.
Avantageusement, la quantité de dérivé phénoliques est de 0,00001 à 10% en poids par rapport au poids total de la composition, de préférence de 0,0001 à 6% en poids et plus particulièrement de 0,01 à 5% en poids.

La composition selon l'invention peut également comprendre une combinaison d'actif. Le second actif peut être soit également sensible à l'eau ou à un pH acide, et sera dans ce cas, également stabilisé dans la phase grasse. Le second actif peut aussi être un actif compatible avec l'eau et/ou à un pH acide et sera introduit dans la phase aqueuse à l'état solubilisé ou dispersé.

La phase grasse selon l'invention doit être choisie de manière à contenir au moins une phase solvante ou huile solvante de l'actif.

Par huile solvante de l'actif, on entend une huile dans laquelle l'actif est solubilisé et stable chimiquement.

Dans le cas où l'actif de la composition selon l'invention est l'ivermectine, l'huile solvante peut être, par exemple, le diisopropyl adipate, le PPG- 15 stearyl ether, l'octyldodecanol, l'oleyl alcool, la triacetine, les caprylic capric triglycerides, le phenoxyethanol, l'alcool benzylique .

D'autres huiles ou corps gras peuvent être ajoutés à la phase grasse solvante de la composition de manière variée par l'homme du métier afin de préparer une composition ayant les propriétés souhaitées, par exemple en consistance ou en texture.

Parmi ces huiles ou corps gras, on peut citer de manière non exhaustive :
- des huiles végétales, comme l'huile d'amande douce vendu par Sictia ou l'huile de sésame vendu par CPF, l'huile de palme, l'huile de soja, l'huile de sésame, l'huile de tournesol, l'huile d'olive.
- des huiles minérales, comme les huiles de paraffine de différentes viscosités comme le Marcol 152®, le Marcol 52® ou le Primol 352® vendu par Esso ;
- des triglycerides comme les triglycerides caprylique / capric vendus sous le nom Mygliol 812® par Sasol,
- des esters, comme l'Octyl Dodecyl Myristate vendu sous le nom de MOD par Gattefosse, le C12-C15 Alkyl benzoate vendu sous le nom de Tegosoft TN par Goldschmit ou l'Isononanoate d'Isononyle vendu sous le nom de DUB ININ par Stéarinerie Dubois, le cetearyl isononanoate vendu sous le nom de Cetiol SN par la société Cognis, le diisopropyl adipate ou crodamol DA vendu par la société CRODA, le palmitate d'isopropyle (crodamol IPP) ou le myristate d'isopropyle (crodamol IPM) vendus par la société CRODA
- des alcools alkoxylés, comme le POE(15) stéaryl éther (stéareth-15), le PPG-15 stéaryl éther benzoate, PPG-5-ceteth-20 ou le POE(20) isohexadecyl éther (isoceteth-20).
- des éthers et dérivés, comme le PPG-15 stearyl ether vendu sous le nom d'Arlamol E® par Croda.
- les alcools de Guerbet comme l'octyldodécanol vendu sous le nom d'Eutanol G® par Cognis
- des huiles animales ou leurs substituts d'origine végétale lorsqu'ils existent, on peut citer la lanoline, le squalène, l'huile de poisson, l'huile de vison avec comme dérivé le perhydrosqualene vendu sous le nom de Sophiderm® par la société Sophim ou bien sous le nom de Cosbiol ® par la société Laserson,
- les polyisobutènes hydrogénés comme le PARLEAM® vendu par la société ROSSOW
- des huiles de silicone, comme la Cyclomethicone vendu sous le nom de ST-Cyclomethicone 5NF® par Dow Corning ou la Dimethicone vendue sous le nom de Q7 9120 silicon fluid® de viscosité de 20 cst à 12500 cst vendue par Dow Corning, ou encore des composés siliconés lipophiles tels que les élastomères polyorganosiloxane tel l'Elastomer 10 commercialisé par Dow Corning.
- et leurs mélanges.

La composition selon l'invention peut également contenir des corps gras de haut point de fusion solides à température ambiante ou encore des gélifiants lipophiles appelés aussi épaississants lipophiles.
Par corps gras de haut point de fusion on entend des composés choisis parmi les cires, les alcools gras, les huiles hydrogénées, les esters d'acides gras.

Par cire, on entend d'une manière générale un composé lipophile, solide à température ambiante (25 °C), à changement d'état solide / liquide réversible, ayant un point de fusion supérieur ou égal à 30 °C pouvant aller jusqu'à 200 °C et notamment jusqu'à 120 °C. Comme cires utilisables, on peut citer la cire de carnauba, les cires microcristallines, la cire d'abeille, commercialisée sous le nom de Cerabeil blanche par Barlocher, ou encore la cire de candellila.

Comme alcools gras utilisables, on peut citer l'alcool oléique, l'alcool cétylique, l'alcool cétearylique ou encore l'alcool stéarylique.

Par huile hydrogénée, on entend les huiles obtenues par hydrogénation catalytique d'huiles animales ou végétales ayant des chaînes grasses, linéaires ou ramifiées, en C₈-C₃₂. Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50®, l'huile de tournesol hydrogénée, l'huile de ricin hydrogénée, commercialisée notamment sous le nom de Cutina HR® par Cognis, l'huile de coprah hydrogénée et l'huile de lanoline hydrogénée,

Comme esters d'acides gras utilisables, on peut citer la lanoline, vendue notamment sous le nom de Medilan® par Croda, les glycérides hydrogénés de coco, vendus sous le nom d'Akosoft 36® par Karlshamns, ou encore le monostéarate de diéthylène glycol ou de propylène glycol, vendus respectivement sous le nom d'Hydrine ou de Monostéol par Gattefossé ou encore le Glyceryl behenate vendu sous le nom de compritol 888® par Gattefossé.

De préférence, la phase grasse comprend au moins une huile solvante de l'actif choisi parmi les triglycérides caprylique/caprique, les huiles minérales ou végétales et les esters. Préférentiellement, les huiles selon l'invention sont le Miglyol 812 (caprylic capric triglycerides), le crodamol DA, et l'huile de paraffine associées à des huiles silicones comme la dimethicone ou des alcools gras.

Ainsi, la phase grasse de l'émulsion selon l'invention peut être présente à une teneur comprise entre 1 et 95 % en poids par rapport au poids total de la composition, de préférence entre 5 et 85%, plus préférentiellement entre 15 et 50% en poids par rapport au poids total de la composition

Les stabilités chimique et physique de la composition selon l'invention sont obtenues notamment par le choix des tensioactifs. Ainsi, la composition selon l'invention comprend également au moins un tensio-actif principal choisi dans la catégorie des esters de sucrose.

Les esters de sucrose sont des tensioactifs non ioniques comprenant un groupe hydrophile constitué par la partie sucrose et un groupe lipophile constitué par un acide gras. Le sucrose ayant généralement un total de 8 groupes hydroxyle, il est donc possible d'obtenir des esters de sucrose allant d'un « mono » ester de sucrose à un « octa » ester de sucrose.
A titre d'exemple non limitatif, d'esters de sucrose, on peut citer le sucrose stearate, le sucrose laurate, ou le sucrose palmitate vendus sous le nom commercial de SURFHOPE par la société MITSUBISHI-KAGAKU, qui sont les esters de sucrose préférés utilisés dans la composition selon l'invention.

Les tensioactifs selon l'invention sont utilisés entre 0.01 et 30 % en poids par rapport au poids total de la composition, préférentiellement entre 0.1 et 15%, et plus préférentiellement entre 0.5.et 7 %.

La composition selon l'invention étant une émulsion huile dans eau, elle comporte une phase aqueuse contenant au moins 5 % d'eau par rapport au poids total de la composition, de préférence entre 5 et 90%.

Dans un mode préféré selon l'invention, la phase aqueuse contient également un polyol (au mininum un triol) de préférence sélectionné parmi le groupe des alcools trihydriques (comme le glycerol), tetrahydriques (comme la diglycerine), hexahydriques (comme le sorbitol).
Ainsi la quantité de polyol selon l'invention est comprise entre 1 et 40% en poids par rapport au poids total de la composition.

Dans un mode préféré, la composition selon l'invention contient de la glycérine à une teneur comprise entre 1 et 20% et une proportion d'eau comprise entre 5% et 90%.

Dans un mode de réalisation particulièrement préféré, la composition selon l'invention comprend également un ou plusieurs gélifiants de phase hydrophiles. A titre d'exemple non limitatif de gélifiants pouvant entrer dans les compositions selon l'invention, on peut citer, l'Acrylates/C10-30 Alkyl Acrylate Crosspolymer vendu sous le nom de Pemulen TR-1® ou Pemulen TR-2® par la société Noveon, les carbomers vendus sous le nom d'Ultrez 20®, d'Ultrez 10®, de Carbopol 1382® ou de Carbopol ETD2020NF®, de carbopol 981 ou encore carbopol 980 par la Société Noveon, les polysaccharides avec à titre d'exemples non limitatifs la gomme de xanthane telle que le Xantural180® vendu par la société Kelco, la gellan gum vendu sous le nom de Kelcogel® par la société Kelco, la gomme guar, la cellulose et ses dérivés tel que la microcrystalline cellulose et carboxymethyl cellulose de sodium vendue sous le nom d'Avicel CL-611® par la société FMC Biopolymer, l'hydroxypropylmethylcellulose en particulier le produit vendu sous le nom de Methocel E4M premium par la société Dow Chemical ou l'hydroxyéthylcellulose, en particulier, le produit vendu sous le nom de Natrosol HHX 250® par la société Aqualon, la famille des aluminium magnésium silicates tel que le Veegum K vendu par la société Vanderbilt ,la famille des polymères acryliques couplés à des chaînes hydrophobes tel que le PEG-150/decyl/SMDI copolymer vendu sous le nom de Aculyn 44® (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)), la famille des amidons modifiés tels que l'amidon de pomme de terre modifié vendu sous le nom de Structure Solanace ou bien leurs mélanges .et les gélifiants de la famille des polyacrylamides tels que le mélange Sodium acryloyldimethyltaurate copolymer / isohexadecane / polysorbate 80 vendu sous le nom Sepineo P 600 ® (ou de Simulgel 600 PHA®) par la société Seppic, le mélange polyacrylamide / isoparaffine C13-14 / laureth-7 comme, par exemple, celui vendu sous le nom de Sepigel 305 par la société Seppic, la famille des carraghénanes en particulier réparties sous quatre grandes familles : κ, λ, β, ω tel que les Viscarin® et les Gelcarin® commercialisés par la société IMCD
A titre de gélifiant préféré, on peut citer les carbomers comme les carbopol 980® ou 981®, les polyacrylamides comme le Sepineo P 600® ou Simulgel 600 PHA®, les polysaccharides comme la gomme de xanthane.
Le gélifiant tel que décrit ci-dessus peut être utilisé aux concentrations préférentielles allant de 0,001 à 15 % et, plus préférentiellement, allant de 0,01 à 5 %.

La composition selon l'invention peut également comprendre des additifs usuellement utilisés dans le domaine pharmaceutique et cosmétique permettant de conférer à la dite préparation des propriétés spécifiques. L'homme de l'art adaptera le choix de ces additifs en fonction de l'effet attendu.

Parmi les additifs on peut citer en exemple, pris seuls ou combinés :
- des antioxydants tel que la vitamine E et ses dérivés, comme le DL alpha Tocopherol ou l'acetate de tocopherol de Roche ; la vitamine C et ses dérivés, comme l'Ascorbyl Palmitate de Roche, le Butylhydroxy toluene vendu sous le nom de Nipanox BHT par Clariant, le métabisulfite de sodium
- des vitamines telles que la vitamine PP ou niacinamide
- des agents apaisants et/ou anti-irritants tels que le PPG-12/SMDI copolymer vendu par la société Bertek pharmaceuticals sous le nom commercial de Polyolprepolymer-2 ou encore de l'acide glycyrrhetinique ou ses dérivés comme par exemple l'Enoxolone vendu par la société Cognis, ou l'acide hyaluronique.
- des lécitines, du cholestérol
- des conservateurs : On peut citer à titre d'exemples de conservateurs le chlorure de benzalkonium, le bronopol, la chlorhexidine, le chlorocrésol et ses dérivés, l'alcool éthylique, le phénoxyéthanol, le sorbate de potassium, la diazolidinylurée, l'alcool benzylique, les parabens, ou leurs mélanges le methyl parabène vendu sous le nom de Nipagin M par Clariant, le Propyl parabène vendu sous le nom de Nipasol par Clariant,
- des acides ou bases tels que l'acide citrique, le sodium citrate, la triethanolamine, l'aminométhylpropanol, le sodium hydroxyde, la diisopropanolamine,
- des chélatants.

Les additifs seront présents au sein de la composition selon l'invention dans des proportions allant de 0 à 20% en poids total de la composition.

La composition selon l'invention concerne donc une composition topique de type émulsion huile dans eau, comprenant :
- au moins un principe actif sensible à l'eau,
- une phase grasse contenant au moins une phase lipophile solvante de l'actif,
- au moins un tensioactif de la famille des sucroesters
- au moins un polyol,
- au moins 5 % d'eau.

La composition selon l'invention concerne donc une composition topique de type émulsion huile dans eau, comprenant :
- au moins un principe actif sensible à l'eau, choisi parmi les lactones macrocycliques,
- une phase grasse contenant au moins une phase lipophile solvante de l'actif,
- au moins un tensioactif de la famille des sucroesters
- au moins un polyol,
- au moins 5 % d'eau.

Selon un mode préféré, la composition selon l'invention concerne une composition topique de type émulsion huile dans eau, comprenant :
- au moins un principe actif sensible à l'eau, choisi parmi les les lactones macrocycliques,
- une phase grasse contenant au moins une phase lipophile solvante de l'actif,
- au moins un tensioactif de la famille des sucroesters
- au moins un polyol,
- au moins un gélifiant,
- au moins 5 % d'eau.

Dans un mode particulièrement préféré, la composition se présente sous forme d'émulsion et compend:
- au moins un actif sensible à l'eau, le dit actif étant sous une forme solubilisée et stable chemiquement dans la phase huileuse,
- une phase lipophile solvante de l'actif,
- au moins un polyol sélectionné parmi le groupe des alcools trihydriques, tétrahydriques et hexahydriques,
- au moins 5% d'eau, caractérisée en ce que la composition est topique et contient au moins un sensioactif, choisi parmi les sucroesters et en ce que ledit actif sensible à l'eau est l'ivermectine.

La présente invention a également pour objet l'utilisation d'une composition selon l'invention pour la fabrication d'un médicament destiné au traitement :
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle,
- des ichtyoses, des états ichtyosiformes, de la maladie de Darrier, des kératodermies palmoplantaires, des leucoplasies et des états leucoplasiformes, du lichen cutané ou muqueux (buccal),
- des affections dermatologiques avec une composante immuno-allergique inflammatoire, avec ou sans trouble de la prolifération cellulaire, notamment le psoriasis cutané, muqueux ou unguéal, le rhumatisme psoriasique, l'atopie cutanée, telle que l'eczéma, ou dermatite atopique, l'atopie respiratoire ou l'hypertrophie gingivale,
- au traitement des pathologies dues au *Demodex folliculorum* et plus particulièrement de la rosacée cutanée ou ophtalmique,
- des proliférations dermiques ou épidermiques bénignes ou malignes, d'origine virale ou non, notamment les verrues vulgaires, les verrues planes l'épidermodysplasie verruciforme, les papillomatoses orales ou florides, le lymphome T,
- des proliférations pouvant être induites par les ultra-violets notamment des épithélioma baso et spinocellulaires,
- des lésions précancéreuses cutanées notamment les kératoacanthomes,
- des dermatoses immunes notamment le lupus érythémateux,
- des maladies immunes bulleuses,
- des maladies du collagène notamment la sclérodermie,
- des affections dermatologiques ou générales à composante immunologique,
- de désordres cutanés dus à une exposition aux rayonnements U.V, du vieillissement de la peau, photo-induit ou chronologique ou des pigmentations et des kératoses actiniques, ou toutes pathologies associées au vieillissement chronologique ou actinique notamment la xérose,
- des troubles de la fonction sébacée notamment l'hyperséborrhée de l'acné, la séborrhée simple ou la dermite séborrhéique,
- des troubles de la cicatrisation ou des vergetures,
- des désordres de la pigmentation, tel l'hyperpigmentation, le mélasma, l'hypopigmentation ou le vitiligo,
- des affections du métabolisme des lipides, tel l'obésité, l'hyperlipidémie, le diabète non insulino-dépendant ou le syndrome X,
- des affections inflammatoires telles que l'arthrite,
- des états cancéreux ou précancéreux,
- de l'alopécie de différentes origines, notamment l'alopécie due à la chimiothérapie ou aux rayonnements,
- des troubles du systèmes immunitaire, tel l'asthme, le diabète sucré de type I, la sclérose en plaque, ou autres disfonctionnements sélectifs du système immunitaire, ou
- des affections du système cardiovasculaire telles que l'artériosclérose ou l'hypertension.

Préférentiellement, la composition selon l'invention, contenant de l'ivermectine sera utilisé pour le traitement de rosacée, la composition selon l'invention contenant de l'hydroquinone, ou du rucinol seront utilisées pour le traitement des désordres de la pigmentation.

Dans un second mode préféré d'utilisation de la composition, celle-ci contiendra du rucinol et sera utilisée pour la fabrication d'un médicament destiné à traiter des désordres de la pigmentation.

Les exemples 2 et 4 jusqu'à 21 montrent de façon non exhaustive des exemples de formulation de la composition selon l'invention ainsi que des résultats de stabilité chimique et physique.

### Stabilité du calcitriol dans diverses huiles solvantes (Example comparative ne pas faisant part de l'invention)

Afin d'optimiser la solubilisation de l'actif à incorporer dans les compositions selon l'invention, des tests de stabilité sont réalisés dans diverses huiles solvantes ;

Les préparations sont préparées selon les proportions suivantes :0.00666% de calcitriol / 0.29629% de BHT" une quantité suffisante d'huile solvante pour obtenir 100%.

| Solvant | Caprylic capric triglycerides | |
|---|---|---|
| % Calcitriol/To | T 1 mois | T 3mois |
| TA | 100.3 | 99.9 |
| | | |

| Solvant | PPG-15 stearyl ether | |
|---|---|---|
| % Calcitriol/To | T 1 mois | |
| TA | 100.00 | |
| | | |

| Solvant | Octyldodecanol | |
|---|---|---|
| % Calcitriol/To | T 1 mois | T 3mois |
| TA | 98.3 | 95.00 |
| | | |

| Solvant | Diisopropyl adipate | |
|---|---|---|
| % Calcitriol/To | T 1 mois | T 3mois |
| TA | 101.5 | 97.2 |

### Exemple 2 - Stabilité de l'hydroquinone dans diverses huiles solvantes

Les préparations sont préparées selon les proportions suivantes :4% d'hydroquinone, une quantité suffisante d'huile solvante pour obtenir 100%.

| Solvant | Caprylic capric triglycerides | |
|---|---|---|
| % hydroquinone/To | T 3 mois | |
| 40°C | 95.00 | |
| | | |

| Solvant | PPG-15 stearyl ether | |
|---|---|---|
| % hydroquinone/To | T 3mois | |
| 40°C | 96.00 | |

| Solvant | Diisopropyl adipate | |
|---|---|---|
| % hydroquinone/To | T 3 mois | |
| | 97.4 | |

### Stabilité de l'Ivermectine dans diverses huiles solvantes (Example comparative ne pas faisant part de l'invention)

Les préparations sont préparées selon les proportions suivantes : 1% d'ivermectine, une quantité suffisante d'huile solvante pour obtenir 100%.

| Solvant | Diisopropyl adapipate | |
|---|---|---|
| % ivermectine/To | T 1 mois | |
| 40°C | 96.7 | |
| | | |

| Solvant | PPG-15 stearyl ether | |
|---|---|---|
| % ivermectine/To | T 1 mois | |
| 40°C | 98.07 | |
| | | |
| | | |

| Solvant | phenoxyethanol | |
|---|---|---|
| % ivermectine/To | T 1 mois | |
| 40°C | 98.6 | |
| | | |

| Solvant | Alcool benzylique | |
|---|---|---|
| % ivermectine/To | T 1 mois | |
| 40°C | 98.6 | |

### Exemple 4 - Stabilité du Rucinol dans diverses huiles solvantes

Les préparations sont préparées selon les proportions suivantes 5% de rucinol, une quantité suffisante d'huile solvante pour obtenir 100%.

| Solvant | Capric caprylic triglycerides | |
|---|---|---|
| % Rucinol (%LC) | T 1 mois | |
| 40°C | 99 | |
| | | |

| Solvant | PPG-15 stearyl ether | |
|---|---|---|
| % Rucinol (%LC) | T 1 mois | |
| 40°C | 95 | |
| | | |

| Solvant | PEG-35 castor oil | |
|---|---|---|
| % Rucinol (%LC) | T 1 mois | |
| 40°c | 96 | |
| | | |

| Solvant | PEG-8 caprylic capric triglycérides | |
|---|---|---|
| % Rucinol (%LC) | T 1 mois | |
| 40°C | 100 | |

Technique de dosage par HPLC contre substance de référence.

### Exemple 5 - Composition selon l'invention avec un dérivé de vitamine D

| **Phases** | **Nom INCI** | **% formulaire** |
|---|---|---|
| A | Sucrose laurate | 0.625 |
| A | Sucrose palmitate | 0.625 |
| A | Eau déminéralisée | 1.25 |
| A | Glycérine | 3.75 |
| B | Calcitriol | 0.009 |
| B | BHT | 0.04 |
| B | Caprylic capric triglycerides | 18.741 |
| B | Methyl paraben | 0.2 |
| C | Eau demineralisée | 73.30 |
| D | acrylamide/sodium acryloydimethyltaurate copolymer | 1.5 |

Spécifications à t0 : pH=6.24
Aspect macroscopique : crème blanche fluide
Aspect microscopique : émulsion très fine

### Stabilité physique :

| Temps→ | T 1 mois | T 2 mois | T 3 mois | T 6 mois |
|---|---|---|---|---|
| Conditions de stabilité↓ | | | | |
| TA | Conforme aux spécifications | Conforme aux spécifications | Conforme aux spécifications | Conforme aux spécifications |
| 40°C | Conforme aux spécifications | Conforme aux spécifications | Conforme aux spécifications | Conforme aux spécifications |

### Stabilité chimique:

*Le temps initial (T0) est considéré à 100%*

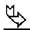 Calcitriol (9 ppm)

| Temps→ | T 1 mois | T 2 mois | T 3 mois | T 6 mois |
|---|---|---|---|---|
| Conditions de stabilité↓ | | | | |
| TA | - | 100.1 | 96.8 | 98.5 |
| 40°C | 96.1 | 98.6 | 99.1 | 104.9 |

### Exemple 6 - Composition selon l'invention avec un dérivé de vitamine D

| **Phases** | **Nom INCI** | **% formulaire** |
|---|---|---|
| A | Sucrose laurate | 2.00 |
| A | Sucrose palmitate | 2.00 |
| A | Eau déminéralisée | 4.00 |
| A | Glycérine | 12.00 |
| B | Calcitriol | 0.009 |
| B | BHT | 0.04 |
| B | Caprylic capric triglycerides | 31.8 |
| B | Huile minérale | 13.50 |
| B | Dimethicone 350 sct | 1.00 |
| B | Cyclomethicone 5 | 13.5 |
| B | Methyl paraben | 0.2 |
| C | Eau demineralisée | 19.551 |
| C | carbomer | 0.1 |
| D | Hydroxyde de sodium (sol 1%) | 0.3 |

**Spécifications à t0 : pH= 5.85**
Aspect macroscopique : crème blanche épaisse
Aspect microscopique : émulsion très fine

### Stabilité physique :

| Temps→ | T 1 mois | T 2 mois | T 3 mois |
|---|---|---|---|
| Conditions de stabilité↓ | | | |
| TA | Conforme aux spécifications | Conforme aux spécifications | Conforme aux spécifications |
| 40°C | Conforme aux spécifications | Conforme aux spécifications | Conforme aux spécifications |

### Stabilité chimique:

*Le temps initial (T0) est considéré à 100%*

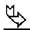 Calcitriol (9 ppm)

| Temps→ | T 1 mois | T 2 mois | T 3 mois |
|---|---|---|---|
| Conditions de stabilité↓ | | | |
| TA | 103.7 | 102.8 | 101.9 |
| 40°C | 101.7 | 100.3 | 97.1 |

### Exemple 7 : Composition avec Ivermectine

| **Phases** | **Nom INCI** | **% formulaire** |
|---|---|---|
| A | Sucrose laurate | 2.5 |
| A | Sucrose stearate | 2.5 |
| A | Eau déminéralisée | 5 |
| A | Glycérine | 15 |
| B | phenoxyethanol | 2.4242 |
| B | Ivermectine | 1.00 |
| B | Diisopropyl adipate | 18 |
| B | Propyl paraben | 0.3030 |
| B | Caprylic capric triglycerides | 53.2728 |

Aspect macroscopique : crème blanche épaisse
Aspect microscopique : émulsion très fine

### Exemple 8 : Composition avec hydroquinone

| **Phases** | **Nom INCI** | **% formulaire** |
|---|---|---|
| A | Sucrose laurate | 0.625 |
| A | Sucrose palmitate | 0.625 |
| A | Eau déminéralisée | 1.25 |
| A | Glycérine | 3.75 |
| B | phenoxyethanol | 0.8 |
| B | hydroquinone | 1.00 |
| B | Diisopropyl adipate | 18.741 |
| B | Methyl paraben | 0.2 |
| C | Eau demineralisée | 71.509 |
| D | acrylamide/sodium acryloydimethyltaurate copolymer | 1.5 |

### Exemple 9 : Composition sous forme de lotion avec Calcitriol

| **Phases** | **Nom INCI** | **% formulaire** |
|---|---|---|
| A | Sucrose laurate | 0.625 |
| A | Sucrose palmitate | 0.625 |
| A | Eau déminéralisée | 1.25 |
| A | Glycérine | 3.75 |
| B | Calcitriol | 0.06 |
| B | BHT | 0.04 |
| B | Caprylic capric triglycerides | 18.741 |
| B | Methyl paraben | 0.2 |
| C | Eau demineralisée | 73.709 |
| D | acrylamide/sodium acryloydimethyltaurate copolymer | 1 |

### Exemple 10 : Composition avec Calcitriol

| **Phases** | **Nom INCI** | **% formulaire** |
|---|---|---|
| A | Decaglycerol monomyristate | 1.8 |
| A | Eau déminéralisée | 1.5 |
| A | Glycérine | 1.5 |
| B | Calcitriol | 0.009 |
| B | BHT | 0.04 |
| B | Caprylic capric triglycerides | 25.401 |
| B | phenoxyethanol | 0.8 |
| B | Methyl paraben | 0.2 |
| C | Glycérine | 5 |
| C | Eau demineralisée | 62.25 |
| D | acrylamide/sodium acryloydimethyltaurate copolymer | 1.5 |

### Exemple 11 : Composition avec Calcitriol

| **Phases** | **Nom INCI** | **% formulaire** |
|---|---|---|
| A | Sucrose laurate | 2.00 |
| A | Sucrose palmitate | 2.00 |
| A | Eau déminéralisée | 4.00 |
| A | Glycérine | 12.00 |
| B | Calcitriol | 0.015 |
| B | BHT | 0.04 |
| B | Caprylic capric triglycerides | 31.80 |
| B | Huile minérale | 13.50 |
| B | Diméthicone 350 sct | 13.50 |
| B | Methyl paraben | 0.20 |
| C | Eau demineralisée | 19.545 |
| D | Carbomer | 0.10 |
| E | Solution soude 10% | 0.30 |

### Exemple 12 - Composition avec calcitriol

| **Phases** | **Nom INCI** | **% formulaire** |
|---|---|---|
| A | Sucrose laurate | 0.825 |
| A | Sucrose stearate | 0.825 |
| A | Eau déminéralisée | 1.65 |
| A | Glycérine | 4.95 |
| B | Calcitriol | 0.009 |
| B | BHT | 0.04 |
| B | Caprylic capric triglycerides | 13.5 |
| B | Alcool cetostearylique | 1.98 |
| B | Huile minérale | 8.333 |
| B | Propyl paraben | 0.10 |
| B | phenoxyethanol | 0.80 |
| C | Eau demineralisée | 60.30 |
| C | Methyl paraben | 0.20 |
| D | Glycérine | 5 |
| E | acrylamide/sodium acryloydimethyltaurate copolymer | 1.5 |

Spécifications à t0 : pH= 5.43
Aspect macroscopique : crème blanche fluide
Aspect microscopique : émulsion très fine

### Stabilité physique :

| Temps→ | T 1 mois | T 2 mois |
|---|---|---|
| Conditions de stabilité↓ | | |
| TA | Conforme aux spécifications | Conforme aux spécifications |
| 40°C | Conforme aux spécifications | Conforme aux spécifications |

### Stabilité chimique:

*Le temps initial (T0) est considéré à 100%*

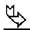 Calcitriol (9 ppm)

| Temps→ | T 1 mois | T 2 mois |
|---|---|---|
| Conditions de stabilité↓ | | |
| TA | 99.9 | 99.2 |
| 40°C | 98.2 | 95.2 |

### Exemple 13 : Composition d'une crème riche avec calcitriol

| **Phases** | **Nom INCI** | **% formulaire** |
|---|---|---|
| A | Sucrose laurate | 1.51 |
| A | Sucrose stearate | 1.51 |
| A | Eau déminéralisée | 3.03 |
| A | Glycérine | 9.09 |
| B | Calcitriol | 0.009 |
| B | BHT | 0.04 |
| B | Caprylic capric triglycerides | 25.32 |
| B | Huile minérale | 24.84 |
| B | Propyl paraben | 0.10 |
| B | phenoxyethanol | 0.80 |
| C | Eau demineralisée | 19.70 |
| C | Methyl paraben | 0.20 |
| E | Simulgel 600 | 0.20 |

Spécifications à t0 : pH= 6.08
Aspect macroscopique : crème blanche épaisse
Aspect microscopique : émulsion très fine

### Stabilité physique :

| Temps→ | T 1 mois | T 2 mois | T 3 mois |
|---|---|---|---|
| Conditions de stabilité↓ | | | |
| TA | Conforme aux spécifications | Conforme aux spécifications | Conforme aux spécifications |
| 40°C | Conforme aux spécifications | Conforme aux spécifications | Conforme aux spécifications |

### Stabilité chimique:

*Le temps initial (T0) est considéré à 100%*

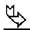 Calcitriol (9 ppm)

| Temps→ | T 1 mois | T 2 mois | T 3 mois |
|---|---|---|---|
| Conditions de stabilité↓ | | | |
| TA | 101.4 | 105.4 | 103.8 |
| 40°C | 104.7 | 104.0 | 100.4 |

### Exemple 14 : Composition avec une combinaision de Calcitriol et de propionate de Clobetasol

| **Phases** | **Nom INCI** | **% formulaire** |
|---|---|---|
| A | Sucrose laurate | 0.625 |
| A | Sucrose palmitate | 0.625 |
| A | Eau déminéralisée | 1.25 |
| A | Glycérine | 3.75 |
| B | Calcitriol | 0.009 |
| B | BHT | 0.04 |
| B | Caprylic capric triglycerides | 18.741 |
| B | Methyl paraben | 0.2 |
| C | Eau demineralisée | 67.30 |
| C | Clobetasol propionate | 0.05 |
| C | Propylène glycol | 8 |
| D | acrylamide/sodium acryloydimethyltaurate copolymer | 1.5 |

### Exemple 15 : composition contenant du Rucinol

| **Phases** | **Nom INCI** | **% formulaire** |
|---|---|---|
| A | Sucrose stearate | 0.825 |
| A | Sucrose palmitate | 0.825 |
| A | Eau déminéralisée | 1.65 |
| A | Glycérine | 4.95 |
| B | rucinol | 1.00 |
| B | Caprylic capric triglycerides | 23.75 |
| B | Methyl paraben | 0.2 |
| C | Eau demineralisée | 65.3 |
| D | acrylamide/sodium acryloydimethyltaurate copolymer | 1.5 |

Spécifications à t0 : pH= 5.46
Aspect macroscopique : crème blanche
Aspect microscopique : émulsion très fine

### Exemple 16 : composition contenant du Rucinol

| **Phases** | **Nom INCI** | **% formulaire** |
|---|---|---|
| A | Sucrose stearate | 2.5 |
| A | Sucrose palmitate | 2.5 |
| A | Eau déminéralisée | 5.00 |
| A | Glycérine | 15.00 |
| B | rucinol | 3.00 |
| B | Caprylic capric triglycerides | 69.97 |

### Exemple 17 : Composition avec Ivermectine

| **Phases** | **Nom INCI** | **% formulaire** |
|---|---|---|
| A | Sucrose laurate | 0.825 |
| A | Sucrose stearate | 0.825 |
| A | Eau déminéralisée | 1.65 |
| A | Glycérine | 4.95 |
| B | phenoxyethanol | 0.8 |
| B | Ivermectine | 0.33 |
| B | Diisopropyl adipate | 5.94 |
| B | Propyl paraben | 0.10 |
| B | Caprylic capric triglycerides | 17.58 |
| C | Eau demineralisée | 65.30 |
| C | Methyl paraben | 0.20 |
| D | acrylamide/sodium acryloydimethyltaurate copolymer | 1.50 |

Aspect macroscopique : crème blanche épaisse
Aspect microscopique : émulsion très fine

### Exemple 18 : composition contenant du Rucinol

| **Phases** | **Nom INCI** | **% formulaire** |
|---|---|---|
| A | Sucrose stearate | 1.00 |
| A | Sucrose palmitate | 2.00 |
| A | Eau déminéralisée | 1.5 |
| A | Glycérine | 9.00 |
| B | rucinol | 2.5 |
| B | Caprylic capric triglycerides | 34.00 |
| B | Methyl paraben | 0.2 |
| C | Eau demineralisée | 48.5 |
| D | acrylamide/sodium acryloydimethyltaurate copolymer | 1.3 |

Spécifications à t0 : pH= 6.15
Aspect macroscopique : crème blanche
Aspect microscopique : émulsion très fine

### Exemple 19 : composition contenant du Rucinol et un rétinoide

| **Phases** | **Nom INCI** | **% formulaire** |
|---|---|---|
| A | Sucrose stearate | 0.825 |
| A | Sucrose palmitate | 0.825 |
| A | Eau déminéralisée | 1.65 |
| A | Glycérine | 4.95 |
| B | rucinol | 1.00 |
| B | Caprylic capric triglycerides | 23.75 |
| B | Methyl paraben | 0.2 |
| C | Eau demineralisée | 60.0 |
| C' | Eau demineralisée | 5.00 |
| C' | adapalène | 0.1 |
| C' | Poloxamer 124 | 0.2 |
| D | acrylamide/sodium acryloydimethyltaurate copolymer | 1.5 |

### Exemple 20 : Mode opératoire des compositions :

- chauffer dans la cuve principale, la phase aqueuse A contenant les tensioactifs et , le cas échant, la totalité ou une partie des polyols à 75°C sous agitation jusqu'à solubilisation complète des tensioactifs
- chauffer dans un récipient séparé, la phase grasse B à 75°C. Rajouter en fin de chauffe l'actif solubilisé, chauffé si nécessaire à 50°C dans une partie de l'huile solvante.
- Lorsque les 2 phases sont à la température de 75°C, procéder à l'émulsification en incorporant progressivement la phase B dans la phase A sous fort cisaillement.
- Laisser ensuite refroidir l'émulsion.sous agitation modérée
- Rajouter progressivement à partir de 40°C, le reste de la phase aqueuse C sous agitation modérée.
- Rajouter ensuite le cas échant les éléments de la phase D toujours sous agitation modérée.
- Rajouter éventuellement avant la phase D, les dispersions des autres actifs (phase C')

### Exemple 21 : Etude de la libération/pénétration des actifs formulés dans les compositions selon l'invention.

L'étude a été réalisé avec une composition selon l'invention avec pour actif le calcitriol. L'objectif de cette étude est d'évaluer la libération et la pénétration de l'actif calcitriol formulé dans une composition selon l'invention et de comparer les résultats à ceux de l'actif formulé dans un onguent classique, de type la pommade Silkis®.
Outre une bonne stabilité de l'actif recherché au sein de la composition selon l'invention, une bonne libération /pénétration de l'actif doit également être atteinte afin de pourvoir distribuer l'actif jusqu'à sa cible, dans le présent cas, la peau. Ceci afin de pouvoir obtenir l'effet thérapeutique recherché.

La libération/pénétration du calcitriol est donc mesuré dans et à travers la peau, in-vivo chez le miniporc, après une application unique de calcitriol et la concentration/quantité de calcitriol dans la peau est corrélée avec la réponse pharmacodynamique.

En effet, l'objectif est d'évaluer si la quantité d'actif présente dans la peau tel que libérée par la composition selon l'invention est suffisante pour générer l'activité pharmacodynamique recherchée pour un effet thérapeutique. Pour cela, une composition selon l'invention contenant calcitriol à la concentration de 9µg/g est comparé à la composition de référence, la pommade Silkis formulé avec 9 µg/g de calcitriol.

Pour chaque surface de peau traitée, les données individuelles suivantes ont été calculées et exprimées dans µg ou en pourcentage de la dose appliquée.
Les paramètres mesurés sont les concentrations de Calcitriol dans :
- Stratum Corneum
- Peau Total
- Mesure de la réponse pharmacodynamique (expression des ARNm de la 24-hydroxylase)
- Corrélation entre les concentrations de calcitriol dans la peau et l'intensité de la réponse pharmacodynamique.

En pourcentage de la dose appliquée

| | | | |
|---|---|---|---|
| Composition onguent 9 ppm | SC | 8,09 | 2,91 |
| | Peau | 5,86 | 1,74 |
| Composition selon l'ex.14 9 ppm | SC | *7,88* | *2,85* |
| | Peau | *5,20* | *1,47* |

Les résultats montrent que les quantités de calcitriol libérées par la composition selon l'invention sont significativement du même ordre que celles libérés par l'onguent. Par ailleurs, il a été démontré une augmentation significative de l'expression de l'ARNm de la 24-hydroxylase, rendant compte de l'activité Vitamine D, pour les deux compositions. Ce qui implique que les quantités délivrées de l'actif par la composition selon l'invention sont suffisantes pour donner une activité thérapeutique.

## Revendications

1. Composition pharmaceutique de type émulsion huile dans eau comprenant :
- au moins un actif sensible à l'eau, le dit actif étant sous une forme solubilisée et stable chimiquement dans la phase huileuse,
- une phase lipophile solvante de l'actif,
- au moins un polyol sélectionné parmi le groupe des alcools trihydriques, tétrahydriques et hexahydriques,
- au moins 5 % d'eau, **caractérisée en ce que** la composition est topique et contient au moins un tensioactif, choisi parmi les sucroesters,
et **en ce que** ledit actif sensible à l'eau est l'ivermectine.

2. Composition selon la revendication 1, **caractérisée en ce qu'**elle comprend au moins un gélifiant.

3. Composition selon la revendication 2, **caractérisée en ce que** le gélifiant est choisi parmi les acrylamides, les carbomers ou les polysaccharides,

4. Composition selon l'une des revendications 1 à 3, **caractérisée en ce que** la phase lipophile solvante de l'actif comprend au moins un corps gras choisi parmi les triglycérides capric/caprylique et l'huile minérale.

5. Composition selon l'une des revendications 1 à 4, **caractérisée en ce que** le sucroester est choisi parmi le sucrose stéarate, le sucrose laurate, le sucrose palmitate, seul ou en mélange.

6. Composition selon l'une des revendications 1 à 5, **caractérisée en ce que** le polyol est la glycérine.

7. Utilisation d'une composition selon l'une quelconque des revendications 1 à 6, pour la fabrication d'un médicament destiné au traitement :
- des affections dermatologiques liées à un désordre de la kératinisation portant sur la différenciation et sur la prolifération notamment les acnés vulgaires, comédoniennes, polymorphes, rosacées, les acnés nodulokystiques, conglobata, les acnés séniles, les acnés secondaires telles que l'acné solaire, médicamenteuse ou professionnelle, des ichtyoses, des états ichtyosiformes, de la maladie de Darrier, des kératodermies palmoplantaires, des leucoplasies et des états leucoplasiformes, du lichen cutané ou muqueux (buccal),
- des affections dermatologiques avec une composante immuno-allergique inflammatoire, avec ou sans trouble de la prolifération cellulaire, notamment le psoriasis cutané, muqueux ou unguéal, le rhumatisme psoriasique, l'atopie cutanée, telle que l'eczéma, la dermite atopique, l'atopie respiratoire ou l'hypertrophie gingivale,
- des proliférations dermiques ou épidermiques bénignes ou malignes, d'origine virale ou non, notamment les verrues vulgaires, les verrues planes l'épidermodysplasie verruciforme, les papillomatoses orales ou florides, le lymphome T, des proliférations pouvant être induites par les ultra-violets notamment des épithélioma baso et spinocellulaires,
- des lésions précancéreuses cutanées notamment les kératoacanthomes, des dermatoses immunes notamment le lupus érythémateux, des maladies immunes bulleuses, des maladies du collagène notamment la sclérodermie, des affections dermatologiques ou générales à composante immunologique,
- des désordres cutanés dus à une exposition aux rayonnements U.V, du vieillissement de la peau, photo-induit ou chronologique ou des pigmentations et des kératoses actiniques, ou toutes pathologies associées au vieillissement chronologique ou actinique notamment la xérose,
- des troubles de la fonction sébacée notamment l'hyperséborrhée de l'acné, la séborrhée simple ou la dermite séborrhéique,
- des troubles de la cicatrisation ou des vergetures,
- des désordres de la pigmentation, tel l'hyperpigmentation, le mélasma, l'hypopigmentation ou le vitiligo,
- des affections du métabolisme des lipides, tel l'obésité, l'hyperlipidémie, le diabète non insulino-dépendant ou le syndrome X,
- des affections inflammatoires telles que l'arthrite,
- des états cancéreux ou précancéreux,
- de l'alopécie de différentes origines, notamment l'alopécie due à la chimiothérapie ou aux rayonnements,
- des troubles du système immunitaire, tel l'asthme, le diabète sucré de type I, la sclérose en plaque, ou autres disfonctionnements sélectifs du système immunitaire, ou
- des affections du système cardiovasculaire telles que l'artériosclérose ou l'hypertension.

8. Utilisation selon la revendication 7, d'une composition pour la préparation d'un médicament destiné à traiter le psoriasis et la dermite atopique.

9. Utilisation selon la revendication 7, d'une composition pour la préparation d'un médicament destiné à traiter les désordres de la pigmentation.

## Patentansprüche

1. Pharmazeutische Zusammensetzung vom Typ einer Öl-in-Wasser-Emulsion umfassend:
- mindestens einen wasserempfindlichen Wirkstoff, wobei der genannte Wirkstoff in einer gelösten Form vorliegt und in der Ölphase chemisch stabil ist,
- eine lipophilische Lösungsmittelphase des Wirkstoffs,
- mindestens ein Polyol ausgewählt aus der Gruppe von dreiwertigen, vierwertigen und sechswertigen Alkoholen,
- mindestens 5% Wasser, **dadurch gekennzeichnet, dass** die Zusammensetzung topisch ist und mindestens ein Tensid enthält, das aus Saccharoseestern ausgewählt wird,
sowie dadurch, dass der genannte wasserempfindliche Wirkstoff Ivermectin ist.

2. Zusammensetzung nach Anspruch 1, **dadurch gekennzeichnet, dass** sie mindestens einen gelierenden Bestandteil umfasst.

3. Zusammensetzung nach Anspruch 2, **dadurch gekennzeichnet, dass** der gelierende Bestandteil aus Acrylamiden, Carbomeren oder Polysacchariden ausgewählt wird.

4. Zusammensetzung nach einem der Ansprüche 1 bis 3, **dadurch gekennzeichnet, dass** die lipophilische Lösungsmittelphase des Wirkstoffs mindestens eine fettige Substanz umfasst, die aus Caprin-/Capryl-Triglyceriden ausgewählt wird und Mineralöl.

5. Zusammensetzung nach einem der Ansprüche 1 bis 4, **dadurch gekennzeichnet, dass** das Saccharoseester aus Saccharosestearat, Saccharoselaurat, Saccharosepalmitat, allein oder in einer Mischung ausgewählt wird.

6. Zusammensetzung nach einem der Ansprüche 1 bis 5, **dadurch gekennzeichnet, dass** das Polyol Glyzerin ist.

7. Verwendung einer Zusammensetzung nach einem der Ansprüche 1 bis 6 zur Herstellung eines Arzneimittels zur Behandlung von:
- dermatologischen Störungen, die mit einer Keratinisierungsstörung in Bezug auf Differenzierung und Vermehrung assoziiert werden, insbesondere Akne vulgaris, komedonale, polymorphe, Akne Rosacea, nodulozystische Akne, Akne conglobata, senile Akne, sekundäre Akne, wie zum Beispiel Sonnenakne, Akne medicamentosa oder Berufsakne, Ichthyosis, ichthyosiforme Zustände, Darier-Krankheit, palmoplantare Keratodermie, Leukoplakie und Leukoplakie-ähnliche Zustände, Lichen der Haut oder der Schleimhaut (bukkal),
- dermatologischen Zuständen mit einer entzündlichen, immunallergischen Komponente mit oder ohne Zellproliferationsstörung, insbesondere Haut-, Schleim- oder Nagelpsoriasis, Schuppenflechte-Rheuma, Hautatopie, wie zum Beispiel Ekzem, atopische Dermatitis, respiratorische Atopie oder gingivale Hypertrophie,
- benignen oder malignen dermalen oder epidermalen Proliferationen, die nichtviralen oder viralen Ursprungs sein können, einschließlich Verruca vulgaris, Verruca plana und Epidermodysplasia verruciformis, sowie orale oder floride Papillomatose, T-Zelle-Lymphom, Proliferationenen, die durch UV induziert werden können, insbesondere im Fall von basocellulären und spinocellulären Epitheliomen,
- präkanzerösen Hautläsionen, insbesondere Keratoakanthome, Immundermatosen, insbesondere Lupus erythematodes, bullöse Autoimmunerkrankungen, Kollagenerkrankungen, insbesondere Sklerodermie, dermatologische oder allgemeine Erkrankungen mit einer immunologischen Komponente,
- Hautstörungen, die durch Exposition gegenüber UV-Strahlung hervorgerufen werden, lichtinduziertes oder chronologisches Altern der Haut, oder aktinische Pigmentierungen und Keratosen, oder sonstige Krankheiten, die mit chronologischem oder aktinischem Altern verbunden sind, insbesondere Xerose,
- Störungen der Talgdrüsenfunktion, insbesondere Acne hyperseborrhea, einfache Seborrhoe oder seborrhoische Dermatitis,
- Wundheilungsstörungen oder Dehnungsstreifen,
- Pigmentstörungen, wie zum Beispiel Hyperpigmentierung, Melasma, Hypopigmentierung oder Vitiligo,
- Fettstoffwechselerkrankungen, wie zum Beispiel Fettleibigkeit, Hyperlipidämie, nicht-Insulin-abhängiger Diabetes oder X-Syndrom,
- Entzündungserkrankungen wie zum Beispiel Arthritis,
- Krebs oder Krebsvorstufen,
- Alopezie unterschiedlichen Ursprungs, insbesondere Alopezie aufgrund von Chemotherapie oder Bestrahlung,
- Störungen des Immunsystems, wie zum Beispiel Asthma, Diabetes mellitus Typ I, Multiple Sklerose oder andere selektive Störungen des Immunsystems, oder
- Herz-Kreislauf-Erkrankungen, wie zum Beispiel Arteriosklerose oder Bluthochdruck.

8. Verwendung nach Anspruch 7 einer Zusammensetzung zur Herstellung eines Arzneimittels zur Behandlung von Psoriasis und atopischer Dermatitis.

9. Verwendung nach Anspruch 7 einer Zusammensetzung zur Herstellung eines Arzneimittels zur Behandlung von Pigmentstörungen.

## Claims

1. An pharmaceutical composition of the oil-in-water emulsion type comprising:
- at least one water-sensitive active ingredient, said active ingredient being in a solubilized form and chemically stable in the oily phase,
- a solvent lipophilic phase of the active ingredient,
- at least one polyol selected from the group of trihydric, tetrahydric and hexahydric alcohols,
- at least 5% water, **characterized in that** the composition is topical and contains at least one surfactant, chosen from sucrose esters,
and **in that** said water-sensitive active ingredient is ivermectin.

2. Composition according to claim 1, **characterized in that** it comprises at least one gelling ingredient.

3. Composition according to claim 2, **characterized in that** the gelling ingredient is chosen from acrylamides, carbomers or polysaccharides.

4. Composition according to one of claims 1 to 3, **characterized in that** the solvent lipophilic phase of the active ingredient comprises at least one fatty substance selected from capric/caprylic triglycerides and mineral oil.

5. Composition according to one of claims 1 to 4, **characterized in that** the sucrose ester is selected from sucrose stearate, sucrose laurate, sucrose palmitate, alone or in a mixture.

6. Composition according to one of claims 1 to 5, **characterized in that** the polyol is glycerin.

7. Use of a composition according to any of claims 1 to 6 for the preparation of a drug intended for treating:
- dermatological disorders associated with a keratinization disorder related to differentiation and proliferation, in particular vulgar, comedonal, polymorphic, rosacea acne, nodulocystic acne, conglobata acne, senile acnes, secondary acnes such as acne caused by exposure to sunlight, caused by medication or occupational acne, ichthyosis, ichthyosiform states, Darrier's disease, palmoplantar keratoderma, leukoplakia and leukoplasiform states, cutaneous or mucosal lichen (buccal),
- dermatological conditions with an inflammatory immunoallergic component, with or without a cell proliferation disorder, in particular cutaneous, mucous or ungual psoriasis, psoriatic rheumatism, cutaneous atopy, such as eczema, atopic dermatitis, respiratory atopy or gingival hypertrophy,
- benign or malignant dermal or epidermal proliferations, of viral origin or not, including common warts, flat warts, verruciform epidermodysplasia, oral or florid papillomatosis, T-cell lymphoma, proliferations that can be induced by U.V., especially basal and squamous cell epithelioma,
- precancerous skin lesions, in particular keratoacanthomas, immune dermatoses, in particular lupus erythematosus, autoimmune bullous diseases, collagen diseases, in particular scleroderma, dermatological or general conditions having an immunological component,
- skin disorders caused by exposure to U.V radiation, photo-induced or chronological aging of the skin, or actinic pigmentations and keratoses, or any pathologies associated with chronological or actinic aging, in particular xerosis,
- sebaceous glands function disorders, in particular acne hyperseborrhea, simple seborrhea or seborrheic dermatitis,
- wound healing disorders or stretch marks,
- pigmentation disorders, such as hyperpigmentation, melasma, hypopigmentation or vitiligo,
- lipid metabolism diseases, such as obesity, hyperlipidemia, non-insulin-dependent diabetes or X syndrome,
- inflammatory conditions such as arthritis,
- cancerous or pre-cancerous states,
- alopecia of different origins, especially alopecia due to chemotherapy or radiation,
- immune system disorders, such as asthma, type I diabetes mellitus, multiple sclerosis, or other selective dysfunctions of the immune system, or
- cardiovascular system disorders such as arteriosclerosis or hypertension.

8. Use according to claim 7, of a composition for the preparation of a drug for treating psoriasis and atopic dermatitis.

9. Use according to claim 7, of a composition for the preparation of a drug for treating pigmentation disorders.
